# EUROPEAN PATENT SPECIFICATION

(11) **EP 2 174 611 B1**
(45) Date of publication and mention of the grant of the patent: **29.12.2010**
(21) Application number: 10152179.7
(22) Date of filing: 15.09.2006
(51) Int. Cl.: A61B 17/72, A61B 17/80

(54) **Intramedullary bone plate with modular head**
Intramedulläre Knochenplatte mit modularem Kopf
Plaque osseuse intramédullaire à tête modulaire

(30) Priority: 20.09.2005 US 231710
(43) Date of publication of application: 14.04.2010
(62) Divisional of application: 06803635.9
(73) Proprietor: DVO Extremity Solutions, LLC, Warsaw, Indiana 46580 (US)
(72) Inventor: Running, Donald, Eli, Warsaw IN 46580 (US); Ondrla, Jeffrey Michael, Warsaw, Indiana 46582 (US); Hunt, III M.D., Thomas R, Birmingham, AL 35223 (US); Churchill M.D., R. Sean, Mequon WI 53092 (US)
(74) Representative: Thomson, Craig Richard

(56) References cited:
- US-A- 5 569 249
- US-B1- 6 358 250

## Description

### Field of the Invention

This invention relates generally to implantable, surgical devices and the method for implantation and, in particular, to an improved surgical device to be used in the internal fixation of fractures to the distal radius and other long bones.

### Background of Invention

There are a variety of surgical devices and methods that are being used to treat fractures of the distal radius. Historically, open reduction and internal fixation (ORIF) of distal radius fractures has been accomplished by the implantation of various types of metallic plates, pegs, wires and screws utilizing a dorsal approach and securing such plates on the dorsal aspect of the radius. Examples of such devices can be found in U.S. Patent Nos. 6,706,046 and 6,730,090. Implanting surgical devices using a dorsal approach is technically easier as critical vascular and soft tissue structure are avoided. A shortcoming and surgical complication caused by the prior art and the corresponding dorsal approach is an increased potential for tendonitis and/or tendon rupture caused by the device thickness and non-uniformity of the adjacent device surface. The prior art also has not been designed to stabilize comminuted fractures of the distal radius in that these devices medial-lateral width is too narrow to adequately span and immobilize fracture sites. Further, the prior arts' use of fixation pegs with set angle orientations does not allow for adequate bone fragment fixation in distal radius fractures.

The state of the art for treating fractures of the distal radius has recently shifted to ORIF utilizing a volar approach. The reason for the shift was the possible elimination of tendonitis and/or tendon rupture that had been experienced with surgical devices implanted dorsally. The shortcoming of the volar approach is the presence of significant soft tissue and vascular anatomy and the resulting technically challenging

implantation procedure of the surgical device. Examples of surgical devices implanted using a volar approach include U.S. Patents Nos.: 6,440,135, 6,364,882, 6,508,819, 6,358,250, 6,893,444, 6,767,351 and 6,712,820. In addition, US 5,569,249 describes a cannulated modular intramedullary nail which includes three connectable sections that are connectable end to end. The assembled nail provides an elongated continuous open ended bore that receives a cannulated fastener. The fastener extends the full length of the longitudinal bore of the assembled three nail section. An upper enlarged head portion of the cannulated fastener grips the proximal nail component. The distal end portion of the cannulated fastener has external threads which engage corresponding internal threads within the bore of the distal nail components. The invention described herein addresses these and other shortcomings of the prior art.

### Summary of the Invention

The present invention provides an intramedullary bone plate with modular head as claimed hereafter. Preferred embodiments are set forth in the dependent claims. There is also provided an intramedullary stem element that is composed of a proximal portion and a distal portion. The exterior surface of the distal portion being configured, for example shaped or dimensioned by machined means, surface treatments or applied three-dimensional surface coatings to enhance bone fixation and rotational stability. The proximal portion being of a smaller circular diameter and having an arced geometry in the sagittal plane, allowing for intramedullary contact and securement.

The bone plate may also include a bone plate head element with the medial side of the bone plate head consisting of a downward angled and outward projecting tab member. This medial tab member sits over the medial side of the radius when the intramedullary stem is properly inserted. Numerous angled, non-threaded and through bone screw and surgical k-wire holes may be located in the bone plate head allowing for fixation of bone fragments and anatomic reconstruction of the fractured distal radius. All bone screw holes are typically located in the lateral and central aspects of the bone plate head, with all surgical k-wire holes preferably being located in the medial tab member. A threaded screw hole with a centerline perpendicular to the top surface of the bone plate head may be located in the sheath recess of the bone plate head.

A neck element rigidly connects the bone plate head to the intramedullary stem. The neck element originates at the most distal end of the intramedullary stem and angles in an upward direction connecting to the most proximal edge or bottom of the bone plate head. The neck element offsets axially and longitudinally in the sagittal plane, the intramedullary stem from the bone plate head.

A sheath element may be attached to the bone plate head. The sheath fits within the sheath recess and may be secured by the sheath screw that engages both the sheath and bone plate head. When in the recess, the sheath covers all of the bone screw heads. The sheath may also include nobs or recesses located on the bottom surface wherein when inserted, the nobs would typically project onto the opposing bone screw head wherein the recesses would receive the bone screw head. These nobs and recesses are intended to substantially inhibit any movement by the screw from its implanted position. The sheath, when joined with the bone plate head, is preferably of minimal overall thickness, thereby giving the invention a low profile and congruent surface for the adjacent contacting soft tissue.

The devices described herein are used for treating distal radius fractures and fractures of similar types in other long bones. Typically the intramedullary stem is inserted into the medullary canal of the bone through the fracture site. The intramedullary stem is then seated and the bone plate head is aligned over the fracture site while ensuring the medial tab member is located over the medial bone fragment. By aligning and seating the bone plate head, the fracture is reduced and buttressed. While maintaining the set position of the fracture, holes may be drilled through the bone plate head into the bone fracture fragments. The drill holes and inserted bone screws are typically at set angles as determined by the bone plate head. Following placement of the bone screws, the sheath may be set into the sheath recess with the bottom surface nobs preferably making contact with the two distal bone screw heads or in the alternative, the bone screw heads preferably projecting into the recesses. The sheath screw typically engages both the sheath and bone plate head and draws the sheath tightly into the sheath recess. Lastly, surgical k-wire may be inserted through the holes located in the medial tab member. The surgical k-wire would be used to secure any bone fracture fragments situated near the medial side of the bone. Following final placement of the surgical k-wire, the free ends may be cut and bent into the wire channel that longitudinal connects the holes located on the top surface of the medial tab member.

### Brief Description of the Drawings

The subject matter which is regarded as the invention is particularly pointed out and distinctly claimed in the claims at the conclusion of the specification. The features and advantages of the invention will be apparent from the following detailed description taken in conjunction with the accompanying drawings, which drawings illustrate several embodiments of the invention.

FIG. 1 shows distal end top perspective view of a device described herein.

FIG. 2 shows a top view of a device described herein.

FIG. 3 shows a lateral view of a device described herein.

FIG. 4 shows a cross-section view of a bone plate head element along line 4-4.

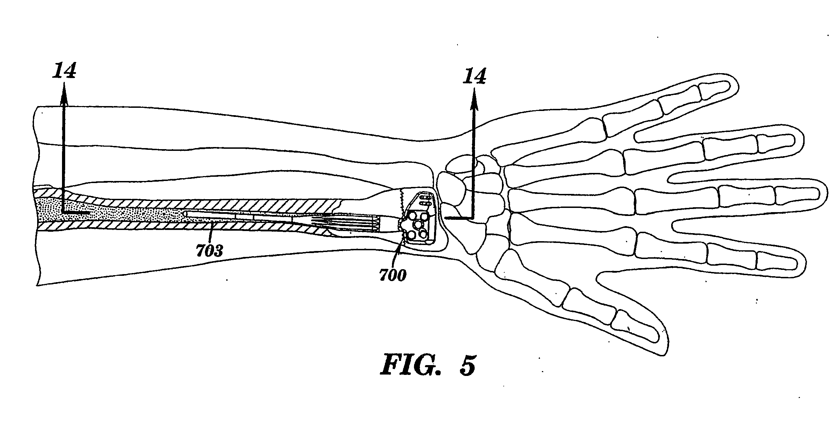

FIG. 5 shows a top view of the radius and ulna with an implanted device described herein.

FIG. 6 shows a cross-section of the distal portion of a intramedullary stem element with longitudinal running flutes along line 6-6.

FIG. 7 shows a distal end top perspective view of a sheath element.

FIG. 8 shows a proximal end bottom perspective view of a sheath element.

FIG. 9 shows a transverse plane section view of distal-medial and distal-lateral bone screw holes.

FIG. 10 shows a sagittal plane section view of distal-medial and distal-lateral bone screw holes.

FIG. 11 shows a side view of a bone screw.

FIG. 12 shows a side view of a sheath screw.

FIG. 13 shows a transverse plane section view of a proximal-lateral bone screw hole.

FIG. 14 illustrates a side view of an implanted device.

FIGS. 15-18 show the method used in treating distal radius fractures with a described device.

FIG. 19 shows a device with a surface coating applied to the intramedullary stem.

FIG. 20 shows an alternative device with porous coating applied to the intramedullary stem element.

FIG. 21 shows a straight proximal portion to the intramedullary stem element.

FIG. 22 is an embodiment of the invention showing an exploded view of the modular bone plate head element and neck element.

FIG. 23 is an alternative embodiment of the invention showing an assembly view of the modular bone plate head element and neck element.

FIG. 24 illustrates a bone plate head element with a proximal edge hood.

FIG. 25 illustrates an assembly view of a straight proximal portion to a intramedullary stem element and a bone plate head element fixed with a neck element angle of ninety degrees.

FIG. 26 illustrates a proximal and bottom perspective of a sheath.

### Detailed Description of the Invention

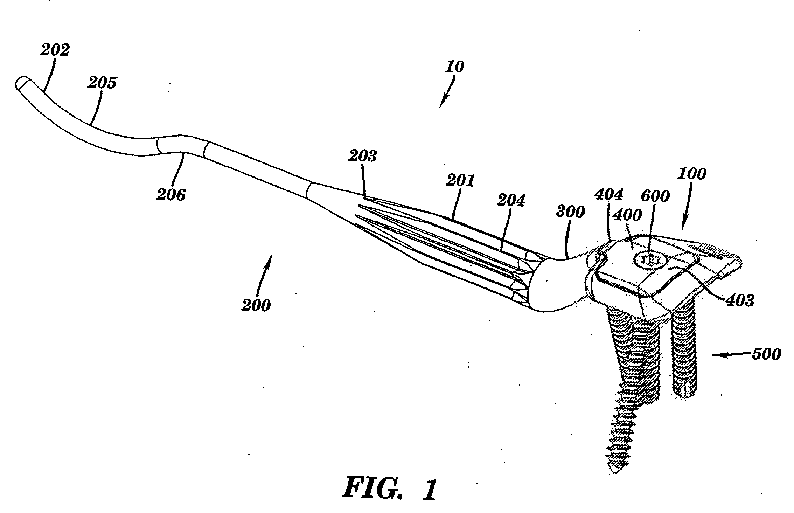

FIG. 1 shows the general arrangement of a intramedullary bone plate with sheath 10. Generally, the intramedullary bone plate with sheath 10 includes a bone plate head 100, an intramedullary stem 200, a connecting neck 300, a sheath 400, a bone screw 500, a sheath screw 600 and surgical k-wire (not shown). The embodiments of the present invention, as described in greater detail below, result in the intramedullary bone plate with with modular head designed to allow for greater intraoperative flexibility and fracture stabilization.

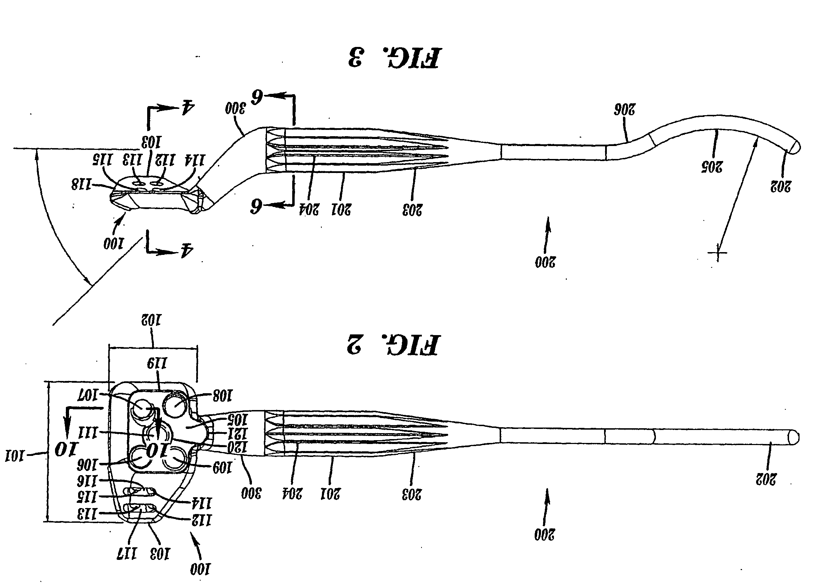

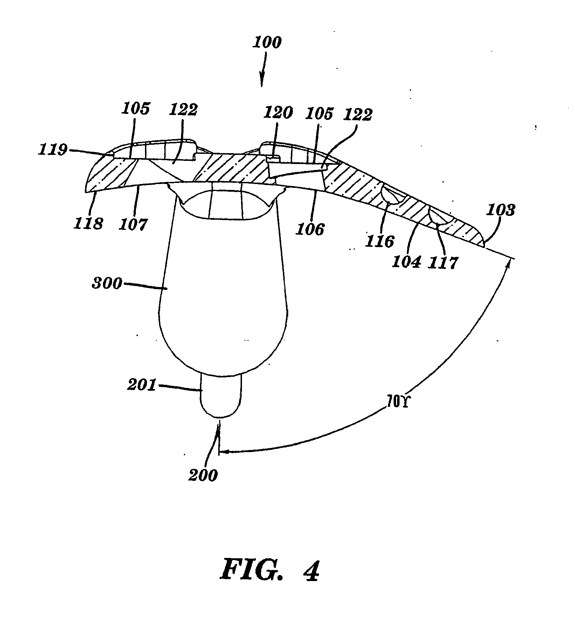

With reference to FIG. 2, the bone plate head 100 is shaped with the overall head width 101 being, preferably, greater than or equal to one half that of the longitudinal length of the head 102. As shown in FIG. 4, the medial tab member 103 is directed downward at an angle of approximately seventy degrees relative to the transverse plane, allowing for increased bone fragment capture. A sheath recess 105 may be oriented in the central aspect of the bone plate head 100 allowing for the insertion of the sheath 400. FIG. 4 also shows the sheath recess 105 including at least one raised boundary 119 on the lateral side of the bone plate head 100. As shown in FIG. 2, the boundary 119 continues around to the proximal side of the bone plate head 100 forming a tab shaped alcove 121 at the neck 3 00 bone plate head 100 junction. FIGS. 2 and 4 also show a raised circular collar 120 being approximately concentric with the threaded screw hole 111. In FIG. 2, three counter-bored non-threaded bone screw holes 106, 107, 109 are located in the central aspect of the bone plate head 100.

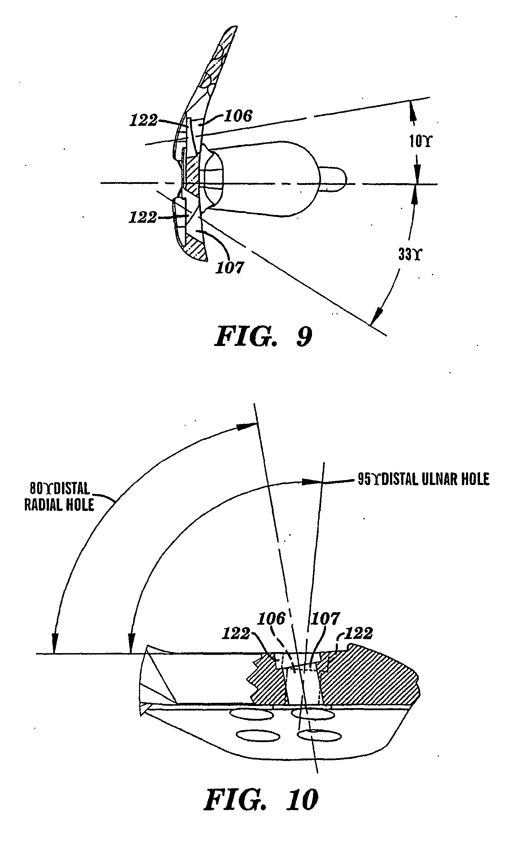

As shown in FIG. 9, the distal-medial screw hole 106 allows for preferably, for example, ten degrees of outward angulation relative to the centerline line of the intramedullary stem 200 and as shown in FIG. 10, the screw hole 106 allows for, preferably, for example, ninety-five degrees relative to the top surface of the bone plate head 100. As shown in FIG. 9, the distal-lateral screw hole 107 allows for, preferably, for example, thirty-three degrees of outward angulation relative to the centerline line of the intramedullary stem 200 and as shown in FIG. 10, the screw hole 107 allows for, preferably, for example, eighty degrees relative to the top surface of the bone plate head 100. The centerline of the proximal-medial screw hole 109 is substantially parallel to the centerline line of the intramedullary stem 200 and about normal to the top surface of the bone plate head 100.

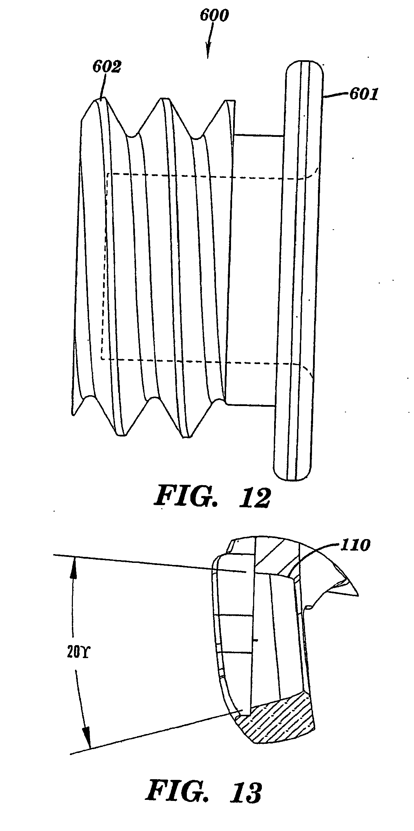

As seen in FIG. 13, the proximal-lateral screw hole 108 may include a spherical seat area 110 allowing for the bone screw 500 to pivot in an inward and outward direction with a preferable overall range of twenty degrees relative to the centerline of the intramedullary stem 200 and a preferable overall range of twenty degrees in the proximal-distal direction. Referring again to FIG. 2, a threaded through hole 111 is preferably located in the central aspect of the sheath recess 105. The centerline of the threaded hole 111 is oriented about normal to the top surface of the sheath recess 105. The sheath screw 600 may be threaded into the threaded hole 111 as described below, following the placement of the sheath 400 into the sheath recess 105.

As shown in FIG. 2 , the medial tab member 103 may include, for example, through holes 112, 113, 114, 115 of smaller diameter relative to bone screw holes 106, 107, 108, 109. Each of the four holes 112, 113, 114, 115 are preferably angled forty-five degrees proximally and fifteen degrees laterally. The medial-distal and medial-proximal holes 114, 115 may be connected by a longitudinal channel 116 and the lateral-distal and lateral-proximal holes 112, 113 may also be connected by a longitudinal channel 117. These longitudinal channels allow for the insertion of surgical k-wire therethrough. FIG. 4 shows the longitudinal channels 116, 117 running substantially parallel to each other and to the centerline of the intramedullary stem 200. Following the insertion of surgical k-wires and securement of bone fragments with these wires, the free ends may pass through bottom surface 104 of the medial tab member 103 and through any of the four holes 112, 113, 114, 115. Dependent upon the exiting hole 112, 113, 114, 115, the free and of the wire may be bent into the adjacent channel 116, 117, with the free end typically being inserted into the corresponding connected hole 112, 113, 114, 115. As illustrated in FIG. 4, the bottom surface of the bone plate head 118 is relatively concave allowing for increased bone-bone plate head contact, while the bottom surface of the medial tab member 103 is typically constructed with a flat geometry.

Referring to FIGS. 1, 2 and 3, the intramedullary stem 200 is comprised of a distal portion 201, a proximal portion 202 and a mid-shaft portion 203. The distal portion 201 is preferably a circular cross-section that then tapers to approximately match and connect to the smaller circular cross-section of the proximal portion 202. FIGS. 1, 2 and 3 show the taper member being located approximately in the mid-shaft portion 203. The taper member usually extends through the mid-shaft portion 203 until it matches the smaller circular diameter of the proximal portion 202.

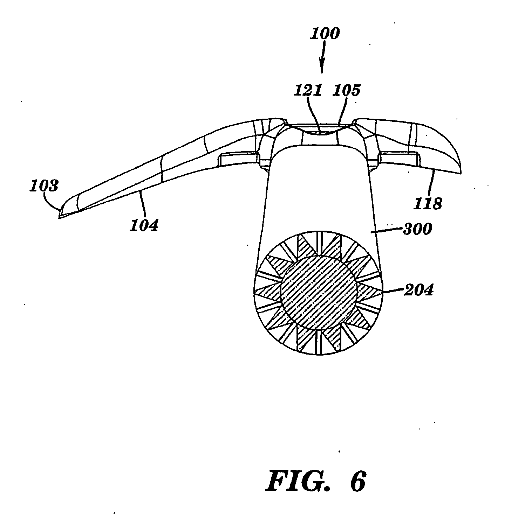

FIG. 6 shows longitudinally running flutes 204 that may be machined into the exterior circumference surface of the distal portion 201. The flutes 204 are shaped and dimensioned preferably for the purpose of medullary canal fixation within the bone and rotational control of the invention 10. As shown in FIGS. 1, 2 and 3, the flutes 204 extend from the most distal end of the distal portion 201 to approximately the mid-point of the taper member in the mid-shaft portion 203.

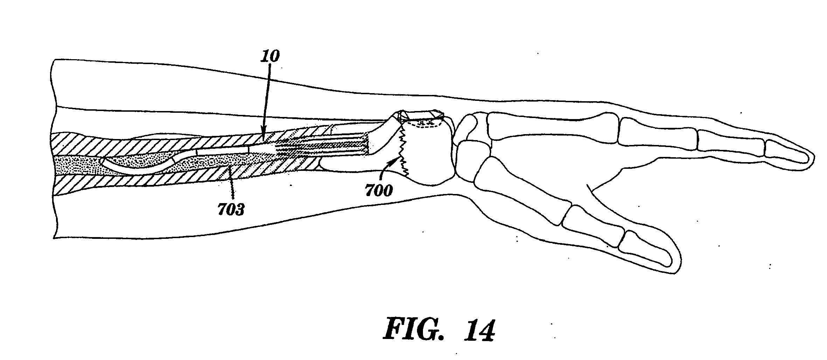

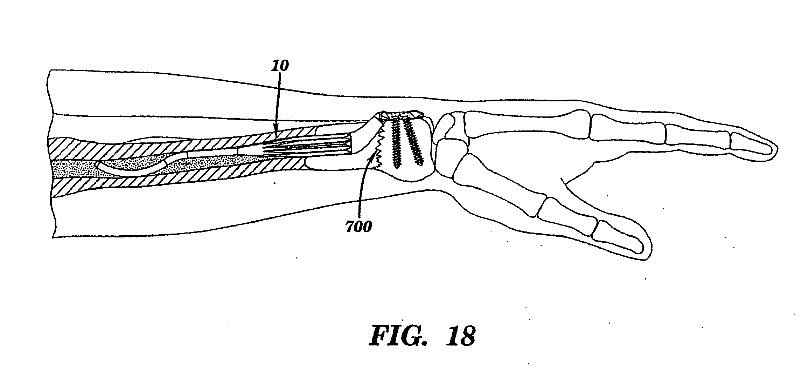

With reference to FIG. 2, the proximal portion 202 is relatively straight with respect to the coronal plane. FIGS. 2 and 3 show the proximal portion 202 being preferably bi-arced with respect to the sagittal plane. As seen again in FIG. 3, the downward projecting arc 205 runs from about the proximal tip of the intramedullary stem 200 to approximately the mid-point of the proximal portion 202. An upward projecting arc 206 of a slightly lesser radius starts approximately at the distal end of the downward arc 205 and extends to about proximal end of the mid-shaft portion 203. As shown in FIGS. 14 and 18, the combination of these two opposing arcs allows for three-point fixation within the bone's medullary canal by the intramedullary stem 200.

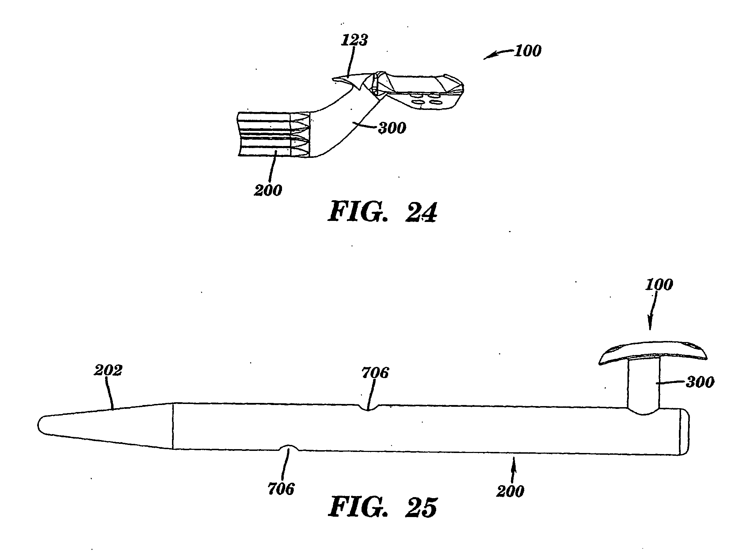

FIG. 14 further shows that implant fixation and stability may be achieved through multiple mechanism, including but not limited to, the three point fixation provided by the intramedullary stem 200, the interference fit of the flutes 204, or a combination of these two mechanisms. Additionally, axial implant fixation and stability is partially achieved by the proximal side of the bone plate head 100 abutting the cortical wall of the distal radius. FIG. 24 shows a device, wherein the entire proximal side of the bone plate head 100 has a distinct hood 123 that projects in the proximal direction making intimate contact with the cortical wall of the distal radius.

FIG. 3 shows the connecting neck element 300 between the most distal end of the intramedullary stem 200 and typically, the proximal edge of the bone plate head 100. Referring again to FIG 3, the neck 300 essentially offsets the intramedullary stem 200 and the bone plate head 100 in two directions. In the sagittal plane, the centerline of the intramedullary stem 200 is substantially parallel to the centerline of the bone plate head 100, while in the coronal and sagittal planes the intramedullary stem 200 is longitudinally offset from the bone plate head 100. As seen in FIG. 3, the neck 300 is typically set at an acute angle relative to the centerline of the intramedullary stem 200, although this angle may reach ninety degrees. With reference to FIGS. 2 and 3, the neck 300 may have a slight reverse taper in that the cross-section of the neck 300 at the bone plate head junction may be smaller relative to the cross-section of the neck 300 at the intramedullary stem junction.

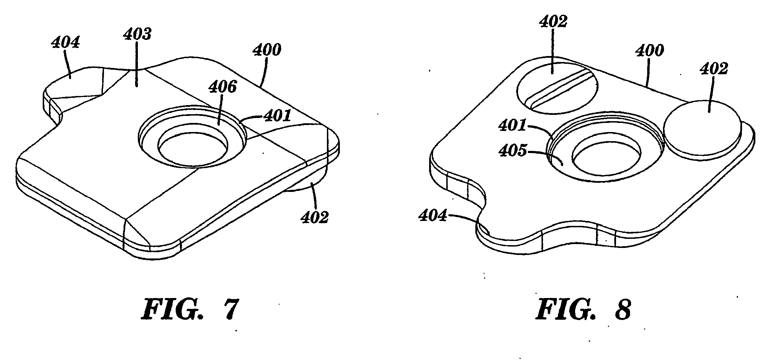

As seen in FIG. 1, the sheath 400 typically has a smooth convex top surface 403 that when inserted into the sheath recess 105 is usually contiguous with the outer aspects of the bone plate head 100. When joined, the bone plate head 100 and the sheath 400 preferably form a low-profile and congruent surface that will allow for non-disruption of the dissected soft-tissue. As seen in FIG. 7, the sheath 400 is essentially a rectangular shape with a tab component 404 extending from the proximal side that may insert into the alcove 121. A threaded through hole 401 is typically located slightly off-center from both the medial-lateral and proximal-distal direction. As shown in FIG. 7, the threaded hole 401 may have a counter-bore 406 to allow for the sheath screw head 601 to sit flush with the top surface 403 when fully engaged. FIG. 1 shows the sheath screw 600 fully inserted. FIG. 8 illustrates the bottom surface of the sheath 400, the nobs 402 and the circular groove 405 that is approximately concentric to the threaded hole 401. Referring again to FIG. 8, the nobs 402 typically are cylinder-like members preferably projecting from the bottom surface of the sheath 400. When the sheath 400 is inserted into the sheath recess 105, the irregular ends of the nobs 402 may project onto the heads of the inserted distal-lateral bone screw 500 and distal-medial bone screw 500 preferably substantially inhibiting any movement of these bone screws 500 from their implanted positions as seen in FIG. 1. Alternatively the sheath 400 may be as shown in FIG. 26, wherein recesses 407 are preferably located on the bottom surface of the sheath 400. In this device when the sheath 400 is inserted into the sheath recess 105, the recesses 407 align with the dome shaped heads (not shown) of the inserted bone screws 500, preferably substantially inhibiting any movement of the bone screws 500 from their implanted positions.

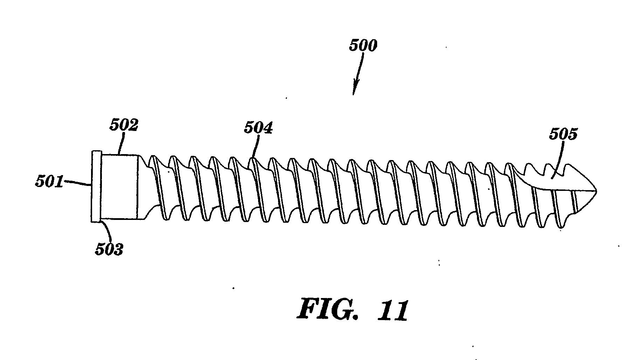

The bone screw 500 as seen in FIG. 11 may be used in conjunction with the bone plate head 100 to secure bone fragments and reduce the distal radial fracture 700. The bone screw 500 is typically available in various lengths. The length of bone screw 500 utilized is usually dependent upon the size and orientation of the bone fragment. The screw head 501 typically has a star shaped indention on the top that matches the insertion tool head (not shown) and is flat. Alternatively, the screw head 501 would be dome shaped (not shown). The screw head 501 has a slight undercut that then transitions into the screw shank 502. The screw shank 502 preferably has a diameter equal to the major diameter of the bone screw 500. The undersurface of the screw head 503 is relatively flat thereby allowing the bone screw 500 to comfortably sit within the counterbore 122 of the bone screw holes 106, 107,109. The threads 504 are machined to allow the bone screw 500 to self-tap.

As seen in FIG. 12, the sheath screw 600 has a predominately flat head 601 and typically has the same a star shaped indention as the bone screw 500. The flat head 601 typically allows for the sheath screw 600, when fully inserted, to sit flush with the sheath top surface 403. The sheath screw 600 when threaded engages both the sheath 400 and the bone plate head 100. Alternatively, the sheath 400 and the bone plate head 100 may be assembled and secured by means other than the sheath screw 600. These other means include but are not limited to, multiple sheath screws, a hinge element fixing the sheath 400 and bone plate head 100 on one side with a snap-like locking element or screw on the opposing side of the hinge element, or a snap-like locking elements on opposing sides of the sheath 400 that may lock within a corresponding opening on the bone plate head 100.

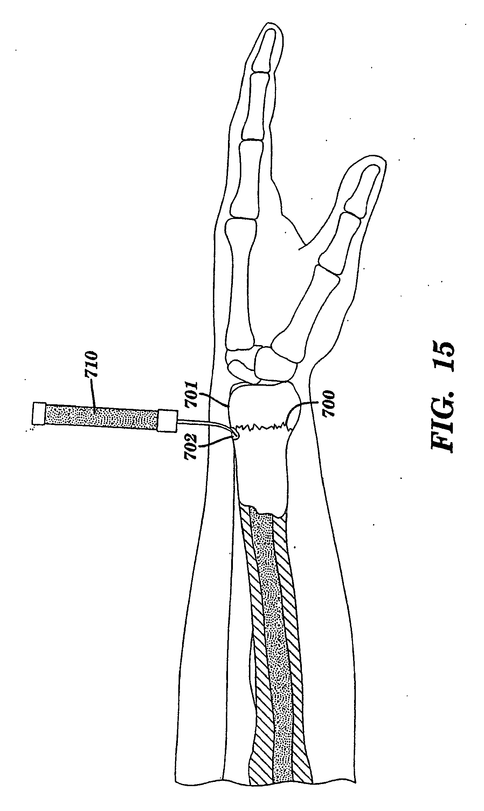

The device 10 may be used to treat distal fractures of the radius 700 and other similar types of fractures in long bones. For distal radius fractures, typically, the implantation method commences with a skin incision being made on the dorsal aspect of the distal radius that is over the 3^{rd} extensor compartment. Several soft tissue structures, including the extensor tendons may be dissected and distracted from the site, with heightened care being taken to protect the radial sensory nerve. The fracture 700 and the involved distal radius are exposed. As shown in FIG. 15, if Lister's tubercule 701 is not already fragmented, a rongeur or other cutting device (not shown) is used to remove the prominence. If access to the medullary canal is not obvious, then an awl 710 is used to prepare an initial opening.

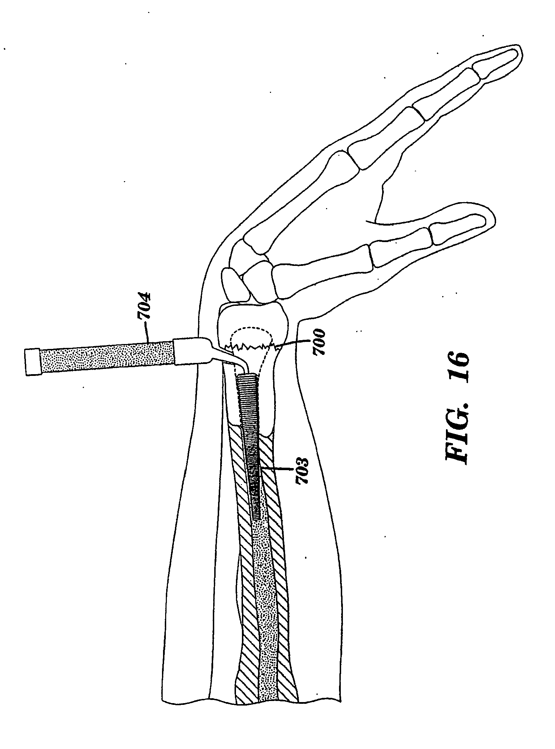

In further preparation of the implant site and the medullary canal 703, FIG. 16 illustrates the use of the one-piece broach 704 that may be inserted into the medullary canal 703 while the wrist is in a relatively flexed position. In the event further seating of the broach is desired, a twist drill may be used to notch the dorsum (not shown).

The intramedullary stem 200 may encounter mild resistance when inserted as it makes contact with the walls of the medullary canal 703. If the distal aspect of the bone plate head 100 overhangs the radiocarpal joint, a notch may be made with a twist drill or rongeur (not shown) in the distal radius allowing the neck 300 to seat further proximally. Once properly placed, the bone plate head 100 will typically be directly under the previous dissected EPL and EDC tendons (not shown) and as a result of the buttressing effect of the seating process and bone plate head 100 placement, the alignment of the fracture 700 should be markedly improved.

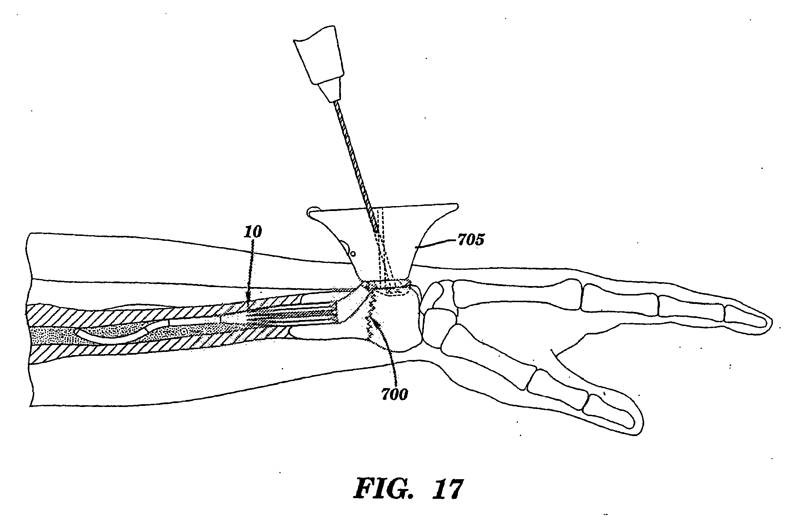

As shown in FIG. 17, following final seating of the intramedullary bone plate device 10 and while maintaining fracture reduction, a drill guide 705 may be attached to the bone plate head 100 for drilling the pilot holes through the bone screw holes 106, 107, 108, 109 in advance of inserting the bone screws 500. A depth gage is typically used (not shown) to determine the appropriate length bone screw 500 to be used when securing the bone fragments.

As shown in FIG. 18, following final tightening of the bone screws 500, the sheath 400 is placed into the sheath recess 105, allowing the nobs 402 or recesses 407 to essentially align with the opposing bone screw heads 501. The sheath 400 is then typically fixed to the bone plate head 100 with the sheath screw 600. In the event further fracture fixation is required, surgical k-wire may be inserted into the wire holes 112,113,114,115 located in the medial tab member 103 with special attention being taken to ensure that the free ends of the surgical k-wire are adequately placed within the wire channels 116,117.

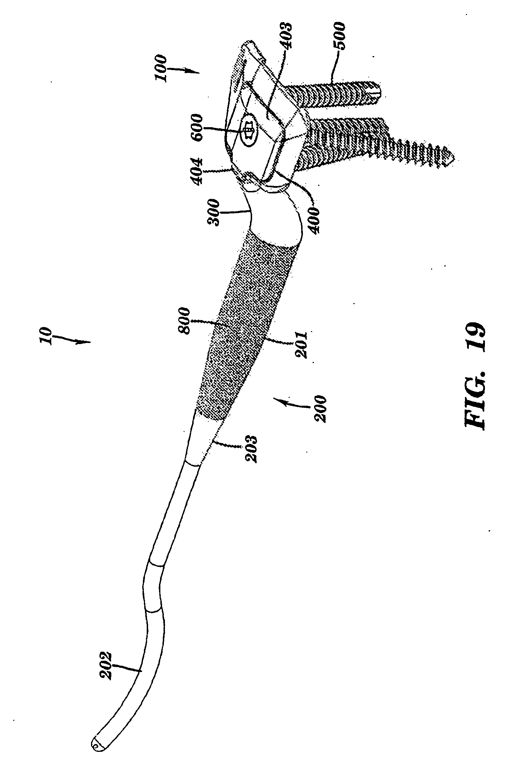

FIG. 19 shows an intramedullary bone plate with sheath 10. As described previously, the distal portion 201 of the intramedullary stem 200 preferably functions to provide medullary canal fixation and rotational stability. In the alternative embodiment of the present invention, the longitudinal flutes 204 are absent from the distal portion 201. The smooth exterior surface of the distal portion 201 may be modified to enhance bone fixation and rotational stability by undergoing a surface treatment 800 that may include, but is not limited to grit blast, in-laid wire mesh and plasma spray.

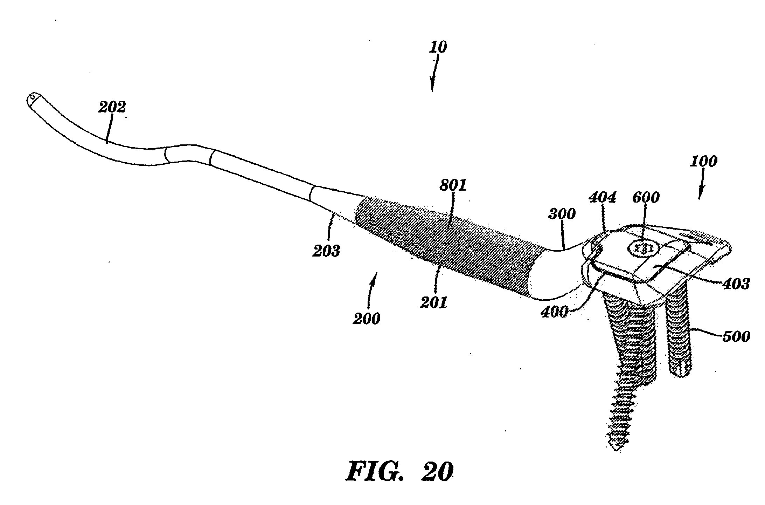

As shown in FIG. 20, the smooth exterior surface of the distal portion 201 may be configured to provide enhanced bone fixation and rotational stability by the application of a three-dimension surface coating 301 that may include, but is not limited to porous-coating and bioactive agents. Such bioactive agents may include, but are not limited to tri-calcium phosphate, hydroxyapatite and bone growth factors.

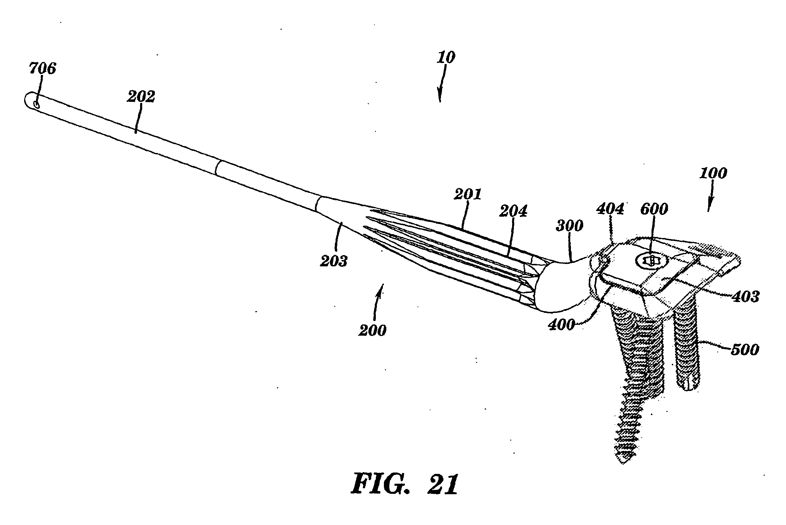

FIG. 21 illustrates an intramedullary bone plate with sheath 10. Referencing FIG. 21 again, the proximal portion 202 of the intramedullary stem 200 is relatively straight in both the sagittal and coronal planes. A transverse through hole 706 is located along the length of the intramedullary stem. An example of this embodiment is seen in FIG. 21 wherein the transverse hole 706 is located near the tip of the intramedullary stem 200. The transverse hole 706 would allow for a pin or screw to be inserted through the cortex of the radius or other long bone in which the invention 10 is implanted. After passing through the transverse hole 706, the pin or screw may be fixed into the diametric opposite outer bone cortex, thereby substantially securing the position of the intramedullary bone plate 10.

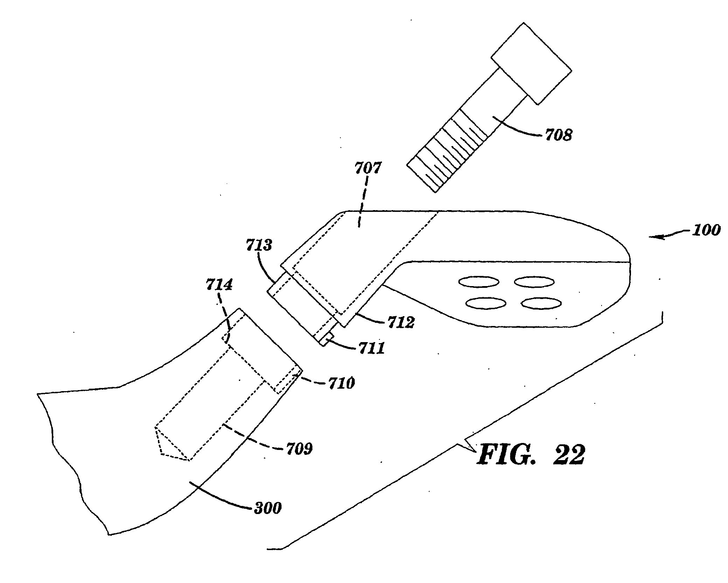

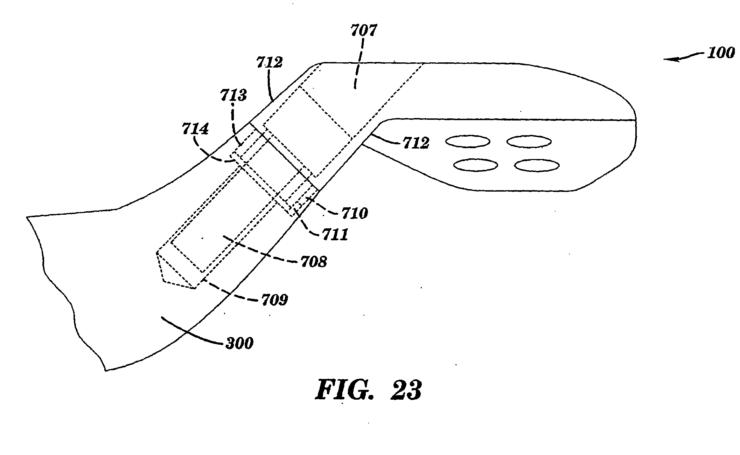

FIGS. 22 and 23 illustrate an embodiment of the present invention, an intramedullary bone plate with sheath 10. The present invention 10 provides for the intramedullary stem 200 and the bone plate head 100 to be connected in a fixed manner by a neck 300. As seen in FIGS. 22 and 23, the bone plate head 100 of the alternative embodiment is modular. On the proximal edge of the bone plate 100, a downward angled connecter 712 may be located with a through hole 707 directed along the connecter's 712 centerline. The connecter end 713 is typically of a smaller diameter relative to the connecter 712, thereby allowing the connecter end 713 to seat within the neck counter-bore 714. To substantially inhibit rotational movement of the bone plate head 100 when the connecter end 713 is inserted and seated in the neck counter-bore 714, a flange 711 may be fixed to one side of the connecter end 713. The flange 711 would typically key into a corresponding notch 710 located in the top aspect of the neck counter-bore 714. A threaded locking screw 708 may then be inserted to engage with a non-through threaded hole 709 located in the neck 300. The centerline of a threaded hole 709 being approximately concentric with the central axis of the neck 300. The preferred location of the threaded hole 709 opening being from the bottom of the neck counter-bore 714 and running to approximately the mid-shaft of the neck 300. An extended sheath (not shown) may be utilized in the alternative embodiment to cover the plurality of bone screw holes 106,107,108,109 and the head locking screw 708. Benefits of having a modular bone plate head 100 include intraoperative customization and inventory flexibility.

FIG. 25 illustrates an intramedullary bone plate with sheath 10. This alternate embodiment may be used for treatment of fractures in bones larger than the radius. As seen in FIG. 25, the intramedullary stem 200 is relatively straight in both the sagittal and coronal planes, with the cross-section of intramedullary stem 200 being substantially circular. The angle which is formed by the neck 300 that may fix the intramedullary stem 200 to the bone plate head 100 is approximately ninety degrees relative to the intramedullary stem 200. As shown in FIG. 25, the proximal portion 202 of the intramedullary stem 200 may be tapered forming a bullet-like shaped end for insertion into the medullary canal of the fractured bone. Additionally, at least one transverse hole 706 is located along the length of the intramedullary stem 200, allowing for the insertion of a pin or screw for implant fixation purpose.

## Claims

1. An intramedullary bone plate device (10), comprising:
an intramedullary stem element (200) configured to provide fixation within a medullary canal of a bone;
a modular bone plate head element (100) having a top surface, a bottom surface, a lateral side, a medial side and a connector element (712), wherein the medial side comprises an outwardly extending member (103), the medial-lateral width of the modular bone plate head element (100) being greater or equal to one half the proximal-distal length of the modular bone plate head element (100), wherein the connector element (712) extends in a downward direction and is fixed to a proximal edge of the modular bone plate head element (100);
a neck element (300) fixed to an end of the intramedullary stem element (200) and configured to couple to the modular bone plate head element (100);
a locking screw (708); and
wherein the locking screw (708) is configured to connect the modular bone plate head element (100) to the neck element (300).

2. The intramedullary bone plate device of claim 1, wherein the connector element (712) comprises a connector end (713), a hole (707), the hole (707) passing thorough the connector (712) and the connector end (713), wherein the hole (707) receives the locking screw (708).

3. The intramedullary bone plate device of claim 2, wherein the connector end (713) further comprises a flange (711), wherein the flange (711) extends radially from the connector end (713) to inhibit rotation of the modular bone plate head element (100) when attached to the neck element (300).

4. The intramedullary bone plate device of claim 1, wherein the neck element (300) extends in an upward direction and further comprises a central axis and a hole (709) having a centerline, wherein the centerline of the hole (709) is concentric with the central axis of the neck element (300).

5. The intramedullary bone plate device of claim 4, wherein the hole (709) further comprises a distal counter-bore (714), the counter-bore (714) being configured to receive and secure the connector end (713) of the connector (712).

6. The intramedullary bone plate device of claim 4, wherein the hole (709) is threaded and threadingly engages the locking screw (708) when the locking screw (708) is inserted through the hole (707) to attach the modular bone plate head element (100) to the neck element (300) upon assembly of the intramedullary bone plate device (10).

7. The intramedullary bone plate device of claim 1, wherein the outwardly extending member (103) is angled in a downward direction and further comprises at least two holes (112,113) (114, 115) completely therethrough, and a longitudinal channel (117) (116) within the top surface of the outwardly extending member (103) connecting the at least two holes.

8. The intramedullary bone plate device of claim 1, wherein the top surface of the modular bone plate head element (100) comprises a sheath recess (105) having a top surface therein.

9. The intramedullary bone plate device of claim 1, wherein the modular bone plate head element (100) further comprises at least one completely therethrough hole (106, 107, 108, 109).

10. The intramedullary bone plate device of claim 8, wherein the modular bone plate head element (100) further comprises a plurality of completely therethrough holes (106, 107, 108, 109, 111) located within the sheath recess (105).

11. The intramedullary bone plate device of claim 10, wherein the plurality of holes:
(a) are longitudinally and laterally (106, 107) displaced within the sheath recess (105);
(b) includes at least one hole (109, 111) having a centerline being about normal to the top surface of the sheath recess (105), and/or;
(c) includes at least one hole (106, 107) having a centerline being angled to the top surface of the sheath recess (105).

12. The intramedullary bone plate device of claims 1 or 11, further comprising a sheath element (400) having a top surface and bottom surface, wherein the sheath element (400) has at least one hole (401) having a centerline being about normal to the top surface of the sheath element (400); further comprising a circular groove (405) in the bottom surface of the sheath element (400) with the circular groove (405) being approximately concentric to at least one completely therethrough hole (401).

13. The intramedullary bone plate device of claim 12, wherein the sheath element (400) has at least one of two raised nobs (402) and recesses (407) fixed to its bottom surface; the sheath element (400) being configured to attach to the modular bone plate head element (100).

14. The intramedullary bone plate device of claims 1, 8, and 12:
(a) wherein the sheath element (400) is configured to be fixed within the sheath recess (105), wherein the sheath element (400) is fixed within the sheath recess (105) with a threaded screw (600),
(b) wherein the sheath element (400) covers said plurality of holes, and/or;
(c) further comprises a means for substantially inhibiting any movement of an inserted bone screw (500), wherein said means comprises a sheath element (400) fixed to a sheath recess (105).

15. The intramedullary bone plate device of claim 1, wherein the intramedullary stem (200) element further comprises a distal portion (201), a mid-shaft portion (203) and a proximal portion (202), wherein:
(a) the distal portion (201) and mid-shaft portion (203) of the intramedullary stem element (200) is straight in the sagittal plane and the proximal portion (202) of the intramedullary stem element (200) is curved in the sagittal plane; and
(b) the diameter of the proximal end of the distal portion (201) tapers in the mid-shaft portion (203) until the diameter matches the smaller diameter of the proximal portion (202) of the intramedullary stem element (200), and/or;
(c) the intramedullary stem element (200) is configured to provide fixation within a medullary canal of a bone, wherein the configuration comprises full circumference, longitudinal flutes (204) extending from and including the distal portion (201) to the mid-shaft portion (203).

## Patentansprüche

1. Eine intramedulläre Knochenplattenvorrichtung (10), die Folgendes beinhaltet:
ein intramedulläres Stammelement (200), das konfiguriert ist, um innerhalb eines Markraums eines Knochens eine Befestigung bereitzustellen;
ein modulares Knochenplattenkopfelement (100) mit einer oberen Oberfläche, einer unteren Oberfläche, einer lateralen Seite, einer medialen Seite und einem Verbinderelement (712), wobei die mediale Seite ein sich nach außen erstreckendes Teil (103) beinhaltet, wobei die medial-laterale Breite des modularen Knochenplattenkopfelements (100) größer oder gleich einer Hälfte der proximaldistalen Länge des modularen Knochenplattenkopfelements (100) ist, wobei sich das Verbinderelement (712) in eine Richtung nach unten erstreckt und an einer proximalen Kante des modularen Knochenplattenkopfelements (100) befestigt ist;
ein Halselement (300), das an einem Ende des intramedullären Stammelements (200) befestigt ist und konfiguriert ist, um mit dem modularen Knochenplattenkopfelement (100) gekoppelt zu sein;
eine Verschlussschraube (708); und
wobei die Verschlussschraube (708) konfiguriert ist, um das modulare Knochenplattenkopfelement (100) mit dem Halselement (300) zu verbinden.

2. Intramedulläre Knochenplattenvorrichtung gemäß Anspruch 1, wobei das Verbinderelement (712) ein Verbinderende (713), ein Loch (707) aufweist, wobei das Loch (707) durch den Verbinder und das Verbinderende (713) läuft, wobei das Loch (707) die Verschlussschraube (708) aufnimmt.

3. Intramedulläre Knochenplattenvorrichtung gemäß Anspruch 2, wobei das Verbinderende (713) ferner einen hervorspringenden Rand (711) beinhaltet, wobei sich der hervorspringende Rand (711) radial von dem Verbinderende (713) erstreckt, um die Drehung des modularen Knochenplattenkopfelements (100), wenn an dem Halselement (300) angebracht, zu unterbinden.

4. Intramedulläre Knochenplattenvorrichtung gemäß Anspruch 1, wobei sich das Halselement (300) in eine Richtung nach oben erstreckt und ferner eine zentrale Achse und ein Loch (709) mit einer Mittellinie beinhaltet, wobei die Mittellinie des Lochs (709) zu der zentralen Achse des Halselements (300) konzentrisch ist.

5. Intramedulläre Knochenplattenvorrichtung gemäß Anspruch 4, wobei das Loch (709) ferner eine distale Senkung (714) beinhaltet, wobei die Senkung (714) konfiguriert ist, um das Verbinderende (713) des Verbinders (712) aufzunehmen und zu sichem.

6. Intramedulläre Knochenplattenvorrichtung gemäß Anspruch 4, wobei das Loch (709) ein Gewinde aufweist und mittels Gewinde mit der Verschlussschraube (708) in Eingriff steht, wenn die Verschlussschraube (708) durch das Loch (707) eingeführt wird, um bei Zusammenbau der intramedullären Knochenplattenvorrichtung (10) das modulare Knochenplattenkopfelement (100) an dem Halselement (300) anzubringen.

7. Intramedulläre Knochenplattenvorrichtung gemäß Anspruch 1, wobei das sich nach außen erstreckende Teil (103) in eine Richtung nach unten abgewinkelt ist und ferner mindestens zwei gänzlich durchgängige Löcher (112, 113) (114, 115) und innerhalb der oberen Oberfläche des sich nach außen erstreckenden Teils (103) einen longitudinalen Kanal (117) (116), der die mindestens zwei Löcher verbindet, beinhaltet.

8. Intramedulläre Knochenplattenvorrichtung gemäß Anspruch 1, wobei die obere Oberfläche des modularen Knochenplattenkopfelements (100) eine Umhüllungsaussparung (105) mit einer oberen Oberfläche darin beinhaltet.

9. Intramedulläre Knochenplattenvorrichtung gemäß Anspruch 1, wobei das modulare Knochenplattenkopfelement (100) ferner mindestens ein gänzlich durchgängiges Loch (106, 107, 108, 109) beinhaltet.

10. Intramedulläre Knochenplattenvorrichtung gemäß Anspruch 8, wobei das modulare Knochenplattenkopfelement (100) ferner eine Vielzahl von gänzlich durchgängigen Löchern (106, 107, 108, 109, 111), welche sich innerhalb der Umhüllungsaussparung (105) befinden, beinhaltet.

11. Intramedulläre Knochenplattenvorrichtung gemäß Anspruch 10, wobei die Vielzahl der Löcher:
a) innerhalb der Umhüllungsaussparung (105) longitudinal und lateral (106, 107) versetzt sind;
b) mindestens ein Loch (109, 111) mit einer Mittellinie, die zu der oberen Oberfläche der Umhüllungsaussparung (105) etwa senkrecht ist, umfasst, und/oder
c) mindestens ein Loch (106, 107) mit einer Mittellinie, die zu der oberen Oberfläche der Umhüllungsaussparung (105) abgewinkelt ist, umfasst.

12. Intramedulläre Knochenplattenvorrichtung gemäß Anspruch 1 oder 11, die ferner ein Umhüllungselement (400) mit einer oberen Oberfläche und einer unteren Oberfläche beinhaltet, wobei das Umhüllungselement (400) mindestens ein Loch (401) mit einer Mittellinie, die zu der oberen Oberfläche des Umhüllungselements (400) etwa senkrecht ist, aufweist; die in der unteren Oberfläche des Umhüllungselements (400) ferner eine kreisförmige Rille (405) beinhaltet, wobei die kreisförmige Rille (405) zu mindestens einem gänzlich durchgängigen Loch (401) annähernd konzentrisch ist.

13. Intramedulläre Knochenplattenvorrichtung gemäß Anspruch 12, wobei das Umhüllungselement (400) mindestens eins von zwei erhöhten Vorsprüngen (402) und Aussparungen (407), die an seiner unteren Oberfläche befestigt sind, aufweist; wobei das Umhüllungselement (400) konfiguriert ist, um an dem modularen Knochenplattenkopfelement (100) angebracht zu werden.

14. Intramedulläre Knochenplattenvorrichtung gemäß Anspruch 1, 8 und 12,
a) wobei das Umhüllungselement (400) konfiguriert ist, um innerhalb der Umhüllungsaussparung (105) befestigt zu werden, wobei das Umhüllungselement (400) mit einer Gewindeschraube (600) innerhalb der Umhüllungsaussparung (105) befestigt ist;
b) wobei das Umhüllungselement (400) die Vielzahl von Löchern abdeckt und/oder
c) die ferner ein Mittel zum Unterbinden von im Wesentlichen jeder Bewegung einer eingeführten Knochenschraube (500) beinhaltet, wobei das Mittel ein Umhüllungselement (400), das an einer Umhüllungsaussparung (105) befestigt ist, beinhaltet.

15. Intramedulläre Knochenplattenvorrichtung gemäß Anspruch 1, wobei das intramedulläre Stammelement (200) ferner einen distalen Abschnitt (201), einen Mittelschaftabschnitt (203) und einen proximalen Abschnitt (202) beinhaltet, wobei:
a) der distale Abschnitt (201) und der Mittelschaftabschnitt (203) des intramedullären Stammelements (200) in der Sagittalebene gerade sind und der proximale Abschnitt (202) des intramedullären Stammelements (200) in der Sagittalebene gekrümmt ist und
b) der Durchmesser des proximalen Endes des distalen Abschnitts (201) in dem Mittelschaftabschnitt (203) verjüngt ist, bis der Durchmesser mit dem kleineren Durchmesser des proximalen Abschnitts (202) des intramedullären Stammelements (200) übereinstimmt, und/oder
c) das intramedulläre Stammelement (200) konfiguriert ist, um innerhalb eines Markraums eines Knochens eine Befestigung bereitzustellen, wobei die Konfiguration vollumlaufende, longitudinale Nuten (204), die sich von dem distalen Abschnitt (201) zu dem Mittelschaftabschnitt (203) erstrecken und den distalen Abschnitt einschließen, beinhaltet.

## Revendications

1. Un dispositif formant plaque osseuse intramédullaire (10), comprenant :
un élément formant tige intramédullaire (200) configuré pour fournir une fixation au sein d'un canal médullaire d'un os ;
un élément formant tête de plaque osseuse modulaire (100) présentant une surface de dessus, une surface de dessous, un côté latéral, un côté médial et un élément formant connecteur (712), où le côté médial comprend un élément s'étendant vers l'extérieur (103), la largeur médiale - latérale de l'élément formant tête de plaque osseuse modulaire (100) étant supérieure ou égale à une moitié de la longueur proximale - distale de l'élément formant tête de plaque osseuse modulaire (100), où l'élément formant connecteur (712) s'étend dans une direction vers le bas et est fixé sur un bord proximal de l'élément formant tête de plaque osseuse modulaire (100) ;
un élément formant col (300) fixé sur une extrémité de l'élément formant tige intramédullaire (200) et configuré pour s'accoupler à l'élément formant tête de plaque osseuse modulaire (100) ;
une vis de verrouillage (708) ; et
où la vis de verrouillage (708) est configurée pour raccorder l'élément formant tête de plaque osseuse modulaire (100) à l'élément formant col (300).

2. Le dispositif formant plaque osseuse intramédullaire de la revendication 1, où l'élément formant connecteur (712) comprend une extrémité de connecteur (713), un trou (707), le trou (707) passant à travers le connecteur (712) et l'extrémité de connecteur (712), où le trou (707) reçoit la vis de verrouillage (708).

3. Le dispositif formant plaque osseuse intramédullaire de la revendication 2, où l'extrémité de connecteur (713) comprend de plus un flasque (711), où le flasque (711) s'étend de façon radiale depuis l'extrémité de connecteur (713) afin d'empêcher la rotation de l'élément formant tête de plaque osseuse modulaire (100) lorsqu'il est attaché sur l'élément formant col (300).

4. Le dispositif formant plaque osseuse intramédullaire de la revendication 1, où l'élément formant col (300) s'étend dans une direction vers le haut et comprend de plus un axe central et un trou (709) présentant une ligne médiane, où la ligne médiane du trou (709) est concentrique avec l'axe central de l'élément formant col (300).

5. Le dispositif formant plaque osseuse intramédullaire de la revendication 4, où le trou (709) comprend de plus un contre-alésage distal (714), le contre-alésage (714) étant configuré pour recevoir et assujettir l'extrémité de connecteur (713) du connecteur (712).

6. Le dispositif formant plaque osseuse intramédullaire de la revendication 4, où le trou (709) est fileté et se met en prise par filetage avec la vis de verrouillage (708) lorsque la vis de verrouillage (708) est insérée au travers du trou (707) pour attacher l'élément formant tête de plaque osseuse modulaire (100) sur l'élément formant col (300) lors de l'assemblage du dispositif formant plaque osseuse intramédullaire (10)

7. Le dispositif formant plaque osseuse intramédullaire de la revendication 1, où l'élément s'étendant vers l'extérieur (103) est coudé dans une direction vers le bas et comprend de plus au moins deux trous (112, 113) (114, 115) le traversant de part en part, et un canal longitudinal (117) (116) au sein de la surface de dessus de l'élément s'étendant vers l'extérieur (103) raccordant les au moins deux trous.

8. Le dispositif formant plaque osseuse intramédullaire de la revendication 1, où la surface de dessus de l'élément formant tête de plaque osseuse modulaire (100) comprend un renfoncement pour gaine (105) dans lequel se trouve une surface de dessus.

9. Le dispositif formant plaque osseuse intramédullaire de la revendication 1, où l'élément formant tête de plaque osseuse modulaire (100) comprend de plus au moins un trou le traversant de part en part (106, 107, 108, 109).

10. Le dispositif formant plaque osseuse intramédullaire de la revendication 8, où l'élément formant tête de plaque osseuse modulaire (100) comprend de plus une pluralité de trous le traversant de part en part (106, 107, 108, 109, 111) situés au sein du renfoncement pour gaine (105).

11. Le dispositif formant plaque osseuse intramédullaire de la revendication 10, où la pluralité de trous :
(a) sont déplacés de façon longitudinale et latérale (106, 107) au sein du renfoncement pour gaine (105) ;
(b) incluent au moins un trou (109, 111) ayant une ligne médiane environ perpendiculaire à la surface de dessus du renfoncement pour gaine (105), et/ou:
(c) incluent au moins un trou (106, 107) ayant une ligne médiane coudée relativement à la surface de dessus du renfoncement pour gaine (105).

12. Le dispositif formant plaque osseuse intramédullaire des revendications 1 ou 11, comprenant de plus un élément formant gaine (400) ayant une surface de dessus et une surface de dessous, où l'élément formant gaine (400) a au moins un trou (401) ayant une ligne médiane environ perpendiculaire à la surface de dessus de l'élément formant gaine (400) ; comprenant de plus une rainure circulaire (405) dans la surface de dessous de l'élément formant gaine (400), la rainure circulaire (405) étant approximativement concentrique à au moins un trou le traversant de part en part (401).

13. Le dispositif formant plaque osseuse intramédullaire de la revendication 12, où l'élément formant gaine (400) a au moins soit deux projections surélevées (402), soit deux renfoncements (407) fixés sur sa surface de dessous ; l'élément formant gaine (400) étant configuré pour s'attacher sur l'élément formant tête de plaque osseuse modulaire (100).

14. Le dispositif formant plaque osseuse intramédullaire des revendications 1, 8, et 12:
(a) où l'élément formant gaine (400) est configuré pour être fixé au sein du renfoncement pour gaine (105), où l'élément formant gaine (400) est fixé au sein du renfoncement pour gaine (105) à l'aide d'une vis filetée (600) ;
(b) où l'élément formant gaine (400) recouvre ladite pluralité de trous, et / ou ;
(c) comprend de plus un moyen destiné à empêcher substantiellement tout déplacement d'une vis à os insérée (500), où ledit moyen comprend un élément formant gaine (400) fixé sur un renfoncement pour gaine (105).

15. Le dispositif formant plaque osseuse intramédullaire de la revendication 1, où l'élément formant tige intramédullaire (200) comprend de plus une portion distale (201), une portion à mi-arbre (203) et une portion proximale (202), où :
(a) la portion distale (201) et la portion à mi-arbre (203) de l'élément formant tige intramédullaire (200) sont droites dans le plan sagittal et la portion proximale (202) de l'élément formant tige intramédullaire (200) est incurvée dans le plan sagittal ; et
(b) le diamètre de l'extrémité proximale de la portion distale (201) s'effile dans la portion à mi-arbre (203) jusqu'à ce que le diamètre corresponde au diamètre plus petit de la portion proximale (202) de l'élément formant tige intramédullaire (200), et / ou ;
(c) l'élément formant tige intramédullaire (200) est configuré pour fournir une fixation au sein d'un canal médullaire d'un os, où la configuration comprend des cannelures longitudinales sur la totalité de la circonférence (204) s'étendant de la portion distale (201), et incluant celle-ci, à la portion à mi-arbre (203).
